Europäisches Patentamt

European Patent Office

Office européen des brevets

(11) Publication number: **0 167 298**
A1

# EUROPEAN PATENT APPLICATION

(21) Application number: 85303982.4

(22) Date of filing: 05.06.85

(51) Int. Cl.⁴: **G 01 N 33/53**
G 01 N 33/543, B 01 L 11/00

(30) Priority: 05.06.84 GB 8414363

(43) Date of publication of application:
08.01.86 Bulletin 86/2

(84) Designated Contracting States:
AT BE CH DE FR GB IT LI NL SE

(71) Applicant: UNILEVER PLC
Unilever House Blackfriars P.O. Box 68
London EC4P 4BQ(GB)

(84) Designated Contracting States:
GB

(71) Applicant: UNILEVER NV
Burgemeester s'Jacobplein 1 P.O. Box 760
NL-3000 DK Rotterdam(NL)

(84) Designated Contracting States:
BE CH DE FR IT LI NL SE AT

(72) Inventor: Prior, Michael Evans
330 Newton Road
Rushden Northants(GB)

(72) Inventor: Dawes, Clive Christopher
78 Sandling Lane
Maidstone Kent(GB)

(74) Representative: Stancliffe, Terence Christopher et al,
UNILEVER PLC Patents Division P.O. Box 68 Unilever
House
London EC4P 4BQ(GB)

(54) Methods and apparatus for carrying out clinical, chemical and biological testing.

(57) A method for carrying out a clinical, chemical or biological test, in which a macro-solid phase adsorbent test material is contacted with a sample and later washed or treated with a washing liquid or reagent liquid, characterised in that the washing or reagent liquid treatment is carried out by (a) inserting the macro-solid phase test material into an apertured container in which there is located a compressible resilient porous liquid-retaining solid which contains washing or reagent liquid, (b) compressing the porous solid while the test material is in its inserted position to the extent necessary to wash or treat the surface of the test material, (c) releasing the compression to allow the porous solid to reabsorb substantially all the washing or treatment liquid, and (d) removing the washed or treated test solid substantially free of the washing or treatment liquid.

- 1 -        P3002


Methods and Apparatus for Carrying Out Clinical, Chemical and Biological Testing

This invention relates to devices and their use in processes of clinical, chemical and biological testing. In particular, the invention relates to devices for the application of liquids, especially for example aqueous washing liquids or reagents, such as water or buffer solutions, to solid adsorbents or absorbent materials which play a part in such test processes.

One important application of the invention is in the facilitation of washing processes which have to be applied to small bodies of adsorbent or absorbent materials during the course of analytical or diagnostic procedures, such as binding or absorption or adsorption tests, especially immunoassays using solid immunosorbents.

It is an aim of the invention to enable a small solid body to be washed by an arrangement for exposing it to washing fluid while containing the washing fluid against much of the normal risk of liquid spillage that attends manipulations of this kind, so that a number of such manipulations can be conveniently carried out by an operator working at a table or bench without personal or environmental contamination by materials present on the solid body or in the wash fluid. It is recognised that complete security against risk of contamination during such liquid treatments is often very hard to achieve. The present invention aims to provide devices that can reduce such contamination risks significantly, even if it is not possible to eliminate them entirely with simple apparatus. The management of such contamination risks is particularly important in connection with diagnostic tests that involve possibly infectious material such as possibly septic clinical samples, or samples containing viral elements.

Among the prior art devices for handling test materials, GB Specification No. 1 392 912 (Philips) describes a device for use in radioimmunoassay in which a syringe contains an elastic sponge of which the porous surface carries a layer of antibody relevant to the assay. In the use of this device, a sample liquid is aspirated, the sponge is incubated with the sample liquid and then squeezed empty, before determination of its radioactivity. This prior art is not concerned with the problem of disposal of possibly contaminated materials used in test procedures, or with the spent washing liquids used in such procedures.

The prior art also includes a number of laboratory washing arrangements, eg. US 3 897 002 and FR 2 177 651. It is considered that these disclosures also do not concern themselves with the problem mentioned above.

According to the invention there is provided a method for carrying out a clinical, chemical or biological test, in which a macro-solid phase adsorbent test material is contacted with a sample and later washed or treated with a washing liquid or reagent liquid, wherein the washing or reagent liquid treatment is carried out  by (a) inserting the macro-solid phase test material into an apertured container in which there is located a compressible resilient porous liquid-retaining solid which contains washing or reagent liquid, (b) compressing the porous solid while the test material is in its inserted position, to the extent necessary to wash or treat the surface of the test material, (c) releasing the compression to allow the porous solid to reabsorb substantially all the washing or treatment liquid, and (d) removing the washed or treated test material substantially free of washing or treatment liquid.

A washer or liquid applicator device according to the invention comprises:

an apertured liquid container, having an aperture of suitable size to allow insertion of a solid test material which is to be washed or to have liquid applied to it;

a compressible resilient porous liquid-retaining solid, located within the container and (in use) retaining a liquid for washing or application to the said solid material, and

a compression member for compressing the porous liquid-retaining solid, while the solid test material is in its inserted position, to the extent necessary to wash the surface of the test material, the arrangement being such that liquid overflow does not occur upon compression of the porous solid, and that on release of the compressive force on the compression member the liquid can return and be reabsorbed by the porous solid.

Suitable examples of arrangements to prevent liquid overflow are (a) provision of a stop or limit to the extent of compression of the porous material, and/or (b) provision of container wells able to contain all the liquid released when the porous material is fully compressed, where the arrangement allows this.

Suitable examples of the apparatus can have one or a plurality of cavities to receive solid test materials in the form of beads, strips, sticks, or pegs, e.g. in the form of assemblies comprising a plurality of solid test materials held by a common holder or carrier.

It is especially suitable for the apparatus to be designed for reasonably close complementarity to the shape and form of whatever test material or assembly of test materials it is desired to wash, and the invention accordingly includes the combinations of wash apparatus and test material as well as the wash apparatus alone.

The test material to be washed may either be completely enclosed in the apparatus during the washing operation or it may partly protrude, e.g. by a holder, with only a relevant sensitised or reactive part being located within the washer.

It is preferred for the lining of the cavity or cavities into which the test material is inserted to be substantially rigid and not to be appreciably deformable or compressible, so that the surface of the test material is not appreciably wiped during the washing or liquid-application.

The cavities may therefore be lined with or consist of one or more apertured sleeves, e.g. of substantially rigid material, to allow insertion and removal of the test material and also the entry and exit of washing liquid. These can be let into apertures in the foam porous material itself. The cavities can in particular take the form of borings or cavities or sleeves in or of a rigid solid component of the apparatus, e.g. a lid or top component, that can for example fit within the container and be optionally sealed thereto with for example flexible sheet sealing material.

Compression of the porous liquid-retaining material is provided for by the construction of the apparatus in any suitable way, e.g. as follows. The container of the apparatus can have a flexible wall or wall portion allowing the porous material to be squeezed and made to release its retained liquid. In this arrangement it is preferable to provide at least a pair of rigid wall portions or rigid container lining portions between which the porous material is compressed: this helps to provide uniform compression when the apparatus is squeezed manually. An internal stop or stops can usefully be provided to ensure that the compression of the porous material is partial rather than complete.

In an alternative and preferred arrangement, compression of the porous material is achieved by pressure on the solid top component of the apparatus carrying the cavities into which the test material is inserted, e.g. downward pressure due to a continuation of motion or exertion of pressure or force in the same direction as that in which the test material is inserted into the apparatus. The top component can for this purpose be a more or less close sliding fit in the container of the apparatus, which can for this purpose be substantially rigid. All parts of the apparatus can be of plastics material, e.g. a thermoformed container and a solid moulded e.g. injection moulded sliding insert carrying the cavity or cavities. The container is in this arrangement preferably given an intermediate stop formation to prevent complete compression of the porous material. Alternatively this stop can be part of the sliding insert, or a separate, (e.g. substantially incompressible) solid component placed within the container.

The available distance of travel of the movable component(s) in the apparatus dictates the volume of liquid released at a maximum compression in use from the porous material. This volume is preferably matched carefully to the free space around the inserted test material so that the released liquid flows around the surfaces to be washed without overflow from the apparatus. If desired, the foam may contain less than the maximum volume of liquid it is capable of retaining.

Recovery of the apparatus from the compressed condition may occur by intrinsic resilience of the porous material or by a separate spring arrangement if desired.

After use the apparatus can be discarded to an incinerator if need be with little or no risk of environmental contamination en route. If desired, a clip-on lid can be provided.

The washing liquid may be any liquid that it is desired to apply to the test material, e.g. water, buffer, or saline, e.g. phosphate buffered saline to wash immunosorbent plastics surfaces, with or without detergents or other additives according to the purpose of the washing operation, and optionally including colour-forming or coloured reagents capable of indicating to the naked eye that the washing operation has been carried out, or other relevant reagents.

The resilient porous liquid-retaining material is chosen from among those materials that can retain liquid without appreciable dripping or loss in the absence of compression: preferably no such loss occurs at all. If the porous solid is to contain and release a reagent, then it can be chosen to be effectively inert with respect to that reagent. If it is to be used to contain washing liquid which will be discarded, it can usefully be chosen as to possess sorptive capacity with respect to materials which are likely to be washed off the test objects. Preferably the porous material is a microporous hydrophilic foam material with a large (>c.90%) void volume. Microporous products suitable for this purpose can for example be made as described in GB Patent Specification No. 1 576 228 (Akzo), and certain suitable materials may also be found amongst those prepared according to GB Patent Specifications No. 1 078 055 (Rohm & Haas), No. 1 428 125 (ICI), No. 1 493 356 (ICI), USP 3 225 127 (Bayer), and European Patent application No. 0 060 138 (Unilever). Also suitable is a hydrophilic polyurethane foam with a water-holding capacity of about 30 times its own weight, and fine pores, about 70 to the linear inch, as available commercially under the name Acquell (Trade Mark) from Scott Paper Company, Chester, Pennsylvania, USA.

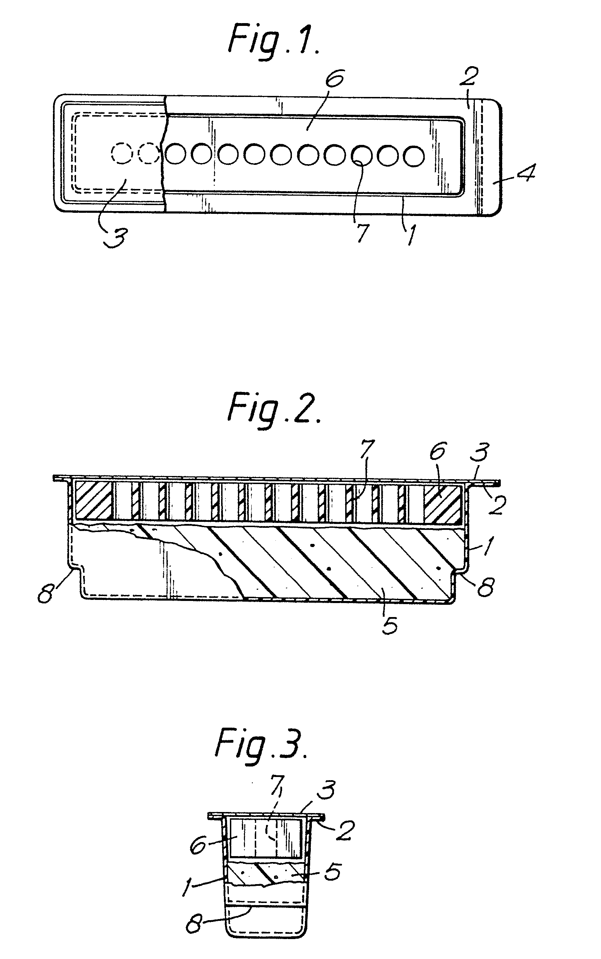

A non-limitative example of the invention is described below with reference to the accompanying drawings in which Figures 1, 2 and 3 respectively show in diagrammatic form a plan, side part-sectional view and end part-sectional view of a washer or liquid applicator arrangement according to an embodiment of the invention.

The apparatus comprises a thermoformed substantially rigid plastics container 1 with a flat upper flange 2 to which is bonded a sealing metal or metallised plastics or plastics foil strip 3 intended to be broken or torn off before use of the washing apparatus by pulling at end 4 where there is a weakened or perforate end to flange 2. The article can be prepared for use with or without a liquid content: either a suitable liquid can be included or it can be left to the user to add liquid after opening the device.

Contained within container 1 is a volume 5 of microporous compressible resilient hydrophilic form which contains and retains a volume of washing liquid, e.g. purified water or substantially isotonic (about 0.9% w/v) saline buffered at near neutral pH. In this case the foam is 'Acquell' (Trade Mark) polyurethane foam. Such material is effectively inert with respect to the test reagents.

Occupying nearly all the remaining volume of container 1 is a sliding insert 6 of solid injection-moulded plastics material having a number of cavities such as 7 in it. These cavities are open at the top and at the bottom, receive the test objects to be washed at the upper opening, and allow inflow and outflow of liquid by the bottom opening.

The pattern of cavities 7 corresponds to the shape of the test articles to be washed, with a little clearance to ensure that as far as possible delicate surfaces are not scraped or rubbed. In this example the test articles to be washed are for example those shown in Figure 3 of published GB Patent Application No. 2 114 738 and described in that specification. Of course, the sliding insert can alternatively be given whatever pattern of cavities is needed to accommodate the objects to be washed and can have if needed some retaining constriction, neck, plug or mesh to stop the objects from dropping right through.

In use the object to be washed can be inserted into the cavities 7 in sliding insert 6 and the insert 6 directly or indirectly pushed down manually until its lower end edges come to rest against shelf-formations 8 in container 1 which act as stops against the further downward movement of insert 6. Foam 5 is compressed by the downward movement and releases part of its liquid content which as free liquid then flows into the cavities 7 in sliding insert 6, and there, without overflowing, contacts and washes the surfaces of the test objects to be washed. On withdrawal, preferably at a moderately slow and defined rate of the test objects or else on stopping the downward pressure, the insert 6 rises back to its original position and the free liquid is drawn back substantially entirely into its original position held in foam 5, from which it does not escape by dripping.

This apparatus enables the washing of possibly infective material, e.g. material possibly contaminated by hepatitis virus, with very low risk of contaminating the user or the environment, because of the good degree of containment and absorption of the washing fluid. Whenever it is arranged that there is no liquid overflow from one cavity 7 to the next the risk of cross-contamination between test objects, i.e. possibly between different samples representing different levels of infective material, is practically eliminated.

This arrangement is especially of use where test objects comprising macro-solid phase specific binding sorbents, e.g. immunosorbents, have to be treated to washing operations in the course of immunoassay procedures. The arrangement can therefore form part of a convenient test kit comprising complementary formed macro-solid phase immunosorbent and ancillary materials to carry out the assays. "Complementary" means only that the immunosorbents can be inserted conveniently into the cavities. It can be seen that the invention disclosed by the present specification and claims is susceptible to variations and modifications to achieve the functions and purposes described above: and includes all combinations of the features described and illustrated herein.

CLAIMS

1.    A method for carrying out a clinical, chemical or biological test, in which a macro-solid phase adsorbent test material is contacted with a sample and later washed or treated with a washing liquid or reagent liquid, characterised in that the washing or reagent liquid treatment is carried out by (a) inserting the macro-solid phase test material into an apertured container in which there is located a compressible resilient porous liquid-retaining solid which contains washing or reagent liquid, (b)  compressing the porous solid while the test material is in its inserted position to the extent necessary to wash or treat the surface of the test material, (c) releasing the compression to allow the porous solid to reabsorb substantially all the washing or treatment liquid, and (d) removing the washed or treated test solid substantially free of the washing or treatment liquid.

2.    A washer or liquid applicator device suitable to form part of a diagnostic test kit which involves the use of a solid adsorbent test material, which device comprises:

an apertured liquid container (1), having an aperture of suitable size to allow insertion of a solid test material which is to be washed  or to have liquid applied to it;
a compressible, resilient porous liquid-retaining solid (5), located within the container and (in use) retaining a liquid for washing or application to the said solid material, and
a compression member (6) for compressing the porous liquid-retaining solid(s), while the solid test material is in its inserted position, to the extent necessary to wash the surface of the test material, the arrangement being such that liquid overflow doe not occur upon compression of the porous solid (5), and that on release of compressive force on the compression member (6) the liquid can return and be reabsorbed by the porous solid (5).

3.  A device according to Claim 2, wherein the compression member (6) comprises a sliding insert located within container (1) and possessing aperture(s) (7) to receive the test objects to be washed or treated with reagent liquid.

4.  A device according to Claim 3, wherein a stop (8) is provided to limit the movement of compression-member (6).

5.  A device according to Claim 1, wherein the compression member (6) comprises a flexible wall of container (1).

6.  A test kit comprising the combination of a washer or liquid applicator device according to Claim 2, 3, 4 or 5, with a complementary solid test body or bodies of adsorbent test material, wherein the solid test body or bodies have such a size as to be receivable with clearance into an aperture or apertures (7) of the washer or liquid applicator device.

7.  A test kit according to Claim 6, wherein the test body carries immunosorbent sensitisation.

8.  A test kit according to Claim 6 or 7, wherein the compression-member (6) comprises a sliding insert having aperture(s) (7) to receive one or a plurality of beads, strips, sticks or pegs of the test body or bodies.

9.  Devices and test kits according to any of Claims 2 to 9, wherein the porous solid (5) is a microporous hydrophilic resilient foam, eg. having at least about 90% void volume.

10. Methods, devices and test kits according to any preceding claim, characterised by any one of more of the several features of the accompanying description and drawings.

0167298

Fig.1.

Fig.2.

Fig.3.

# EUROPEAN SEARCH REPORT

European Patent Office

0167298
Application number

EP 85 30 3982

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| A | AU-B- 495 838 (N.V. PHILIPS' GLOEILAMPENFABRIEKEN) & GB - A - 1 392 912 (Cat. D) | | G 01 N 33/53<br>G 01 N 33/543<br>B 01 L 11/00 |
| A | FR-A-2 536 313 (BIOMERIEUX) | | |
| A | WO-A-8 203 690 (PHARMACIA DIAGNOSTICS AB) | | |
| A | DE-B-1 207 116 (ABBOTT LABORATORIES) | | |
| A | US-A-4 087 248 (L.E. MILES) | | |
| D,A | EP-A-0 060 138 (UNILEVER N.V.) | | TECHNICAL FIELDS SEARCHED (Int Cl.4)<br><br>G 01 N<br>B 01 L |

The present search report has been drawn up for all claims

| Place of search | Date of completion of the search | Examiner |
|---|---|---|
| THE HAGUE | 24-09-1985 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82